(19) **Europäisches Patentamt / European Patent Office / Office européen des brevets**

(11) **EP 2 552 196 B1**

(12) **EUROPEAN PATENT SPECIFICATION**

(45) Date of publication and mention of the grant of the patent:
**09.05.2018 Bulletin 2018/19**

(21) Application number: **11766455.7**

(22) Date of filing: **29.03.2011**

(51) Int Cl.:
*A01N 25/08* (2006.01)  *A01N 25/12* (2006.01)
*A01N 59/00* (2006.01)  *A01N 31/00* (2006.01)
*A01N 25/04* (2006.01)  *A01N 25/34* (2006.01)
*A01N 59/20* (2006.01)  *B82Y 5/00* (2011.01)
*A01N 25/28* (2006.01)

(86) International application number:
**PCT/US2011/030305**

(87) International publication number:
**WO 2011/126832 (13.10.2011 Gazette 2011/41)**

(54) **MULTIFUNCTIONAL SILICA-BASED COMPOSITIONS AND GELS, METHODS OF MAKING THEM, AND METHODS OF USING THEM**

MULTIFUNKTIONELLE ZUSAMMENSETZUNGEN UND GELE AUF SILIKABASIS, VERFAHREN ZU DEREN HERSTELLUNG UND VERFAHREN ZU DEREN VERWENDUNG

COMPOSITIONS ET GELS MULTIFONCTIONNELS À BASE DE SILICE, LEURS PROCÉDÉS DE FABRICATION, ET LEURS PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2010 US 319037 P**

(43) Date of publication of application:
**06.02.2013 Bulletin 2013/06**

(73) Proprietor: **University Of Central Florida Research Foundation, Inc.**
**Orlando, FL 32826 (US)**

(72) Inventor: **SANTRA, Swadeshmukul**
**Orlando, FL 32825 (US)**

(74) Representative: **Tollett, Ian**
**Williams Powell**
**Staple Court**
**11 Staple Inn Buildings**
**London, WC1V 7QH (GB)**

(56) References cited:
**US-A1- 2004 067 247     US-A1- 2010 015 236**

• **KIM, Y. H. ET AL.: 'Preparation and characterization of the antibacterial Cu nanoparticle formed on the surface of Si02 nanoparticles' J. PHYS. CHEM. B vol. 110, 2006, pages 24923 - 24928, XP055096566**
• **BARIK, T. K. ET AL.: 'Nanosilica-from medicine to pest control' PARASITOL. R ES. vol. 103, 2008, pages 253 - 258, XP019630948**

EP 2 552 196 B1

**Description**

BACKGROUND

The worldwide citrus industry is currently battling with two potentially devastating diseases: citrus greening, also known as Huanglongbing or HLB, and citrus canker.

Citrus greening is the most destructive, highly infectious disease of most commercial citrus varieties. This disease is caused by the Gram-negative HLB bacterium that belongs to the genus *Candidatus Liberibacter* as reported by J. M. Bove in "Huanglongbing: A destructive, newly-emerging, century-old disease of citrus," Journal of Plant Pathology 2006, 88, (1), 7-37. HLB threatens the citrus industry worldwide and may cause damage to the citrus industry and economy in the State of Florida. The problem can be severe and kill citrus trees. The disease causes fruits to taste bitter and become deformed, small-sized and poorly-colored, making it unusable and unmarketable. Currently, there is no cure for the HLB.

The Asian Citrus Psyllid (ACP) is an invasive phloem-feeding insect that causes serious damage to citrus plants and citrus plant relatives. Burned tips and twisted leaves result from an infestation of ACP on new growth. In addition, ACP is a vector of HLB and carries HLB and can rapidly spread the disease from one grove to another.

Integrated pest management strategies that include the use of disease-free nursery trees, rapid removal of symptomatic trees and aggressive control of ACPs using foliar insecticides have been the primary focus to manage HLB infected groves according to A. Morris et al. in "Economic tradeoffs of citrus greening management" Citrus Industry 2008, 89 (4), 26-28 and P. L. Hollis in "Scientists combining efforts to combat greening in Florida's citrus industry" Southeast Farm Press 2008, 35 (15), 35.

Unfortunately, the use of foliar insecticides appears to be the only solution available to growers these days to prevent HLB infection, even though such integrated practices are expensive and labor extensive.

Citrus canker is another serious disease, caused by the bacterium *Xauthomouas axouopodis pv. citri,* that affects most commercial citrus varieties and has caused economic loss world-wide according to T. R. Gottwald in "The citrus canker epidemic in Florida: The scientific basis of regulatory eradication policy for an invasive species," Phytopathology 2001, 91, (1), 30-34. Canker causes necrotic lesions on various parts of trees that include fruits, leaves and stems. The severity of the disease or infection is manifested as defoliation, premature fruit drop, blemished fruit and general tree decline as reported by J. H. Graham in "Xauthomouas axonopodis pv. citri: factors affecting successful eradication of citrus canker," Molecular Plant Pathology 2004, 5, (1), 1-15 and A. K. Das in "Citrus Canker-A review," J. Appl. Hort. 2003, 5 (1), 52-60.

While there is no cure for the HLB disease, canker losses have been controlled by the use of appropriate antibacterial agents such as copper (Cu) based compounds, including, but not limited to, Cu oxychloride, Cu sulphate, Cu hydroxide, Cu oxide, ammonia-Cu carbonate, antibiotics, such as, streptomycin, tetracycline, and induced systemic resistance compounds, including, acibenzolar-S-methyl, harpin protein. To date, copper (Cu) has been the gold standard for controlling citrus canker disease worldwide due to its effectiveness in protecting against the possibility of infection and minimal development of Cu resistance by a pathogen.

Due to destructive nature of HLB and citrus canker diseases, there is a need to find solutions to combat HLB and citrus canker diseases.

KIM, Y. H. ET AL.: "Preparation and characterization of the antibacterial Cu nanoparticle formed on the surface of Si02 nanoparticles", J. PHYS. CHEM. B, vol. 110, 2006, pages 24923-24928, XP055096566, discloses the formation of silicon dioxide nanoparticles with copper metal being deposited only on the surface of the nanoparticles. No forms of copper other than copper metal are disclosed. The antibacterial properties of the resulting nanoparticles are discussed.

US 2010/0015236 A1 (MAGDASSI et al) discloses a redispersible powder and aqueous dispersions of nanoparticles of water insoluble organic pesticides. Methods for preparing and using the pesticides are disclosed.

SUMMARY

Aspects of the present invention include a composition as claimed in claim 1, a gel as claimed in claim 11, and methods as claimed in claims 14, 15, 16 and 20. Briefly described, embodiments of this disclosure, among others, include compositions, gels, methods for synthesizing multifunctional silica based nanoparticle gel, method of treating, preventing, or both treating and preventing, a disease in a plant species, method for simultaneously treating citrus plants for citrus canker and preventing the invasion of an Asian Citrus Psyllid (ACP) vector that carries the pathogen and spreads the citrus greening disease in citrus plants, and the like.

BRIEF DESCRIPTION OF THE FIGURES

Fig. 1a is a plot of gas chromatography-mass spectroscopy (GC-MS) data of DMDS (control).

Fig. 1b is a plot of gas chromatography-mass spectroscopy (GC-MS) data of a chloroform extract of DMDS-CuSiNG.

Fig. 2 is a plot of gas chromatography-mass spectroscopy (GC-MS) data of a chloroform extract of DMDS-SiNG, no copper ions.

Fig. 3a is a plot of gas chromatography-mass spectroscopy (GC-MS) data of a chloroform extract of DMDS added to dry CuSiNG lypholized powder with characteristic DMDS peaks shown.

Fig. 3b is a plot of gas chromatography-mass spectroscopy (GC-MS) data of a chloroform extract of DMDS added to dry SiNG as a control showing no DMDS peaks.

DETAILED DESCRIPTION

Before the present disclosure is described in greater detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims. Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure. Any recited method can be carried out in the order of events recited or in any other order that is logically possible.

Embodiments of the present disclosure will employ, unless otherwise indicated, techniques of chemistry, botany, biology, and the like, which are within the skill of the art.

The following examples are put forth so as to provide those of ordinary skill in the art with a complete disclosure and description of how to perform the methods and use the probes disclosed and claimed herein. Efforts have been made to ensure accuracy with respect to numbers (*e.g.,* amounts, temperature, etc.), but some errors and deviations should be accounted for. Unless indicated otherwise, parts are parts by weight, temperature is in °C, and pressure is at or near atmospheric. Standard temperature and pressure are defined as 20 °C and 1 atmosphere.

Before the embodiments of the present disclosure are described in detail, it is to be understood that, unless otherwise indicated, the present disclosure is not limited to particular materials, reagents, reaction materials, manufacturing processes, or the like, as such can vary. It is also to be understood that the terminology used herein is for purposes of describing particular embodiments only, and is not intended to be limiting. It is also possible in the present disclosure that steps can be executed in different sequence where this is logically possible.

It must be noted that, as used in the specification and the appended claims, the singular forms "a," "an," and "the" include plural referents unless the context clearly dictates otherwise. Thus, for example, reference to "a compound" includes a plurality of compounds. In this specification and in the claims that follow, reference will be made to a number of terms that shall be defined to have the following meanings unless a contrary intention is apparent.

Definitions and abbreviations:

Si is used herein to mean silicon dioxide, which is also commonly known as "silica."

NG stands for "Nanogel", which is the gel-like substance formed by the interconnection of nanoparticles, for example, the interconnection of multifunctional silica based nanoparticles.

NP stands for "Nanoparticle", which can have a particle size (*e.g.,* diameter for spherical or substantially spherical nanoparticles) of about 10 to 500 nm, about 10 to 250 nm, about 10 to 100, or about 10 nm to 50 nm. The diameter can be varied from a few nanometers to hundreds of nanometers by appropriately adjusting synthesis parameters, such as amounts of silane precursor, amounts of hydrolyzing agents, polarity of reaction medium, and the like.

CuSiNP stands for copper loaded silica nanoparticle.

CuSiNG stands for copper loaded silica nanogel.

HCuSiNG stands for hybrid Cu-loaded silica nanogel, where the SiNG matrix is loaded with a second silane compound to achieve uniform or substantially uniform plant surface coverage.

Kocide® 3000 pesticide/bactericide; Kocide is a registered trademark of E.I. du Pont de Nemours and Company.

"Uniform plant surface coverage" refers to a uniform and complete (*e.g.,* about 100%) wet surface due to spray application of embodiments of the present disclosure. In other words, spray application causes embodiments of the present disclosure to spread throughout the plant surface.

"Substantial uniform plant surface coverage" refers to about 70%, about 80%, about 90%, or more uniform plant surface coverage.

"Substantially covering" refers to covering about 70%, about 80%, about 90%, or more, of the leaves and branches of a plant.

"Plant" refers to trees, plants, shrubs, flowers, and the like as well as portions of the plant such as twigs, leaves, stems, and the like. In a particular embodiment, the term plant includes a fruit tree such as a citrus tree (*e.g.,* orange tree, lemon tree, lime tree, and the like).

The terms "alk" or "alkyl" refer to straight or branched chain hydrocarbon groups having 1 to 12 carbon atoms, preferably 1 to 8 carbon atoms, such as methyl, ethyl, n-propyl, i-propyl, n-butyl, i-butyl, t-butyl, pentyl, hexyl, heptyl, n-octyl, dodecyl, octadecyl, amyl, 2-ethylhexyl, and the like. Alkyl can include alkyl, dialkyl, trialkyl, and the like.

As used herein, "treat", "treatment", "treating", and the like refer to acting upon a disease or condition with a multifunctional silica based nanoparticle or gel of the present disclosure to affect the disease or condition by improving or altering it. In addition, "treatment" includes completely or partially preventing (*e.g.,* about 70% or more, about 80% or more, about 90% or more, about 95% or more, or about 99% or more) a plant form acquiring a disease or condition. The phrase "prevent" can be used instead of treatment for this meaning. "Treatment," as used herein, covers one or more treatments of a disease in a plant, and includes: (a) reducing the risk of occurrence of the disease in a plant predisposed to the disease but not yet diagnosed as infected with the disease (b) impeding the development of the disease, and/or (c) relieving the disease, *e.g.,* causing regression of the disease and/or relieving one or more disease symptoms.

The term "antibacterial" refers to a compound or composition that destroys bacteria, suppresses or prevents bacteria growth, and/or suppresses, prevents or eliminates the ability of bacteria to reproduce.

The term "antifungal" refers to a compound or composition that destroys fungus, suppresses or prevents fungus growth, and/or suppresses, prevents or eliminates the ability of the fungus to reproduce.

Discussion:

Embodiments of the present disclosure include multifunctional silica based nanoparticles, methods of making multifunctional silica based nanoparticles, multifunctional silica based nanoparticle gels, methods of making multifunctional silica based nanoparticle gels, methods of using methods of making multifunctional silica based nanoparticle and methods of making multifunctional silica based nanoparticle gels, methods of treating plants, and the like. Embodiments of the present disclosure provide for a composition that can be used for multiple purposes. Embodiments of the present disclosure are advantageous in that they can slowly release one or more agents that can be used to prevent or substantially prevent and/or treat or substantially treat a disease or condition in a plant, act as an antibacterial and/or antifungal, and/or act as a repellent to certain types of insects. Another advantage of an embodiment of the present disclosure is that the agent(s) can be controllably released over a long period of time (*e.g.,* from the day of application until a few weeks or months (*e.g.,* about 6 or 8 months)). Embodiments of the present disclosure and features and advantages of these embodiments will be discussed in further detail herein.

Embodiments of the multifunctional silica based nanoparticle include a first component and a second component. In addition, embodiments of the present disclosure can include a multifunctional silica based nanoparticle gel that includes multifunctional silica based nanoparticles. The multifunctional silica based nanoparticle and/or multifunctional silica based nanoparticle gel can be included in a composition to be administered (*e.g.,* spraying) to a plant. The multifunctional silica based nanoparticle gel includes multifunctional silica based nanoparticles inter-connected in an amorphous silica material. The nanoparticles can be inter-connected covalently (*e.g.,* through -Si-O-Si- bonds), physically associated via Van der Waal forces, and/or through ionic interactions (*e.g.,* positively charged copper ions and negatively charged silica nanoparticles). The first and second components can be within the amorphous material as well as in the multifunctional silica based nanoparticle.

The first component can function as an antibacterial and/or antifungal, specifically, treating, substantially treating, preventing or substantially preventing, plant diseases such as citrus greening (HLB) and citrus canker diseases.

The first component (*e.g.,* Cu) can be released from the multifunctional silica based nanoparticle or gel so that it can act as an antibacterial and/or antifungal for a period of time (*e.g.,* from application to days to months). The second component functions as a repellant for insects that can harm plants and/or carry bacteria, diseases, fungi, and the like, that can harm plants (*e.g.,* fruit tree). The second component (*e.g.,* a sulfur compound) of the multifunctional silica based nanoparticle or gel can act as an Asian Citrus Psyllid (ACP) repellant for a period of time (*e.g.,* from application to days to months). Embodiments of the present disclosure have multifunctional purposes to combat diseases in plants such as trees, bushes, and the like, for example, treating citrus greening and citrus canker diseases.

In an embodiment, the release rate of the first and/or second component can be controlled so that characteristics of one or both can be effective for time frames of days to weeks or to months. In other words, the first component and/or second component can be released from the multifunctional silica based nanoparticle or gel starting from the day of application and continuing release to about a week, about a month, about two months, about three months, about four months, about five months, about six months, about seven month, or about eight months.

The multifunctional silica based nanoparticle includes a core and shell. The core includes silica loaded with a first type of first component (*e.g.,* Cu ions). The silica shell that can support a second type of first component (*e.g.,* copper oxide), while also including the first component (*e.g.,* Cu ions). Although not intending to be bound by theory, in an embodiment the second component can interact with the first component (*e.g.,* Cu ions) via a charge-transfer type of interaction, where interaction between the first component and the second component can occur in the nanoparticle and/or in the amorphous silica material.

In an embodiment, the first component can include a copper component, a zinc component, a titanium component, a cerium component, a magnesium component, a zirconium component, polyethyleneimine (PEI), a carbon component (*e.g.,* mixed carbon or soot), fullerene, carbon nanotubes, and a combination thereof. Specifically, the first component can include a copper ion, metallic copper (Cu), copper salt, copper complex, a zinc ion, metallic zinc (Zn), zinc oxide, zinc salt, a silver ion, metallic silver (Ag), silver salt, silver complex, a titanium ion, titanium dioxide ($TiO_2$), a cerium ion, cerium oxides, a magnesium ion, magnesium oxide, a zirconium ion, zirconium oxide, and a combination thereof.

In an embodiment, the copper component can include a copper ion, metallic copper, copper oxide, copper oxychloride, copper sulfate, copper hydroxide, and a combination thereof. The copper component can include copper ions that are electrostatically bound to the silica nanoparticle core or amorphous silica matrix, copper covalently bound to the hydrated surface of the nanoparticle or amorphous silica matrix, and/or copper oxides and/or hydroxides bound to the surface of the nanoparticle or amorphous silica matrix. In an embodiment, the multifunctional silica based nanoparticle and/or gel includes the copper component in two or in all three of these states.

In an embodiment, the copper component can be in a soluble (amorphous) and an insoluble (crystalline) form. By controlling the soluble and insoluble ratio, the release rate of the copper component can be controlled as a function of time. As a result, the release rate of the copper component can be controlled so that antibacterial and/or antifungal characteristics can be effective for time frames of days to weeks or to months. In other words, the copper component can be released from the multifunctional silica based nanoparticle or gel staring from the day of application and continuing release to about a week, about a month, about two months, about three months, about four months, about five months, about six months, about seven month, or about eight months. The ratio of the soluble to insoluble copper component can be adjusted to control the release rate. In an embodiment, the ratio of the soluble copper to the insoluble copper (*e.g.,* Chelated Cu)$_X$ (Crystalline Cu)$_{1-X}$) can be out 0:1 to 1:0, and can be modified in increments of about 0.01 to produce the ratio that releases the Cu for the desired period of time. Parameters that can be used to adjust the ratio include: solvent polarity and protic nature (*i.e.,* hydrogen bonding capability), Cu precursor (*e.g.,* Cu sulfate) concentration, temperature, concentration of silane precursor (such as tetraethylorthosilicate, TEOS), and the like.

In an embodiment, the second component can include a sulfur compound. The sulfur compound does not react or reacts very little (*e.g.,* at such a low percentage or at such a slow rate that the first and second components can still function in a manner and for a time frame described herein) with the first component. The sulfur compound can include alkyl sulfides, alkyl disulfides, alkyl trisulfides, alkyl tetrasulfides, analogues of each, and a combination thereof, where alkyl can include alkyl, dialkyl, and trialkyl. In particular, the sulfur compound can include compounds dimethyl disulfide (DMDS), dimethyl sulfide, diethyl disulfide, diethyl trisulfide, diethyl tetrasulfide, and a combination thereof. The release rate of the second component can be controlled to release starting from the day of application to about a week, about a month, about two months, about three months, about four months, about five months, about six months, about seven month, or about eight months.

In an embodiment, the sulfur compound is DMDS. It should be noted that the sulfur compound can be used as a ACP repellant and is attractive strategy to control the HLB. It should be noted that DMDS is toxic to insects because it disrupts cytochrome oxidase system of the mitochondria and is considered a strong repellent to ACP. DMDS can interact with the first component (*e.g.,* Cu ion) in a charge-transfer type of interaction. Thus, by controlling the amount of first component that the DMDS can interact with, the amount of the first component can be used to control the amount of DMDS present in the multifunctional silica based nanoparticle or gel. Although not intending to be bound by theory, sulfur (electronegative element, polarizable) in DMDS is weakly bound to copper ions (type of ion-dipole interaction). Once Cu is released from the product, DMDS will mostly release as there is no other strong interaction between DMDS and silica nanoparticle/nanogel matrix other than Van der Waals force.

In an embodiment, the amorphous silica gel has no ordered (*e.g.,* defined) structure (opposite to crystalline structure) so an "amorphous gel" refers to gel material having amorphous structural composition. In an embodiment, the number of multifunctional silica based nanoparticle in a gram of multifunctional silica based nanoparticle gel can be difficult to accurately determine. However, the following provides some guidance.

Let's assume amorphous silica gel (completely dehydrated) including about 10 nm size (diameter) inter-connected particles as our test material. One could roughly estimate number of particles per gram of material in the following way:

$$\text{Mass (m) of a single particle} = \text{density of the particle (d)} \times \text{volume of the particle (v)}$$

$$D = 2.648 \text{ gm/cm}^3 \text{ (approx)}$$

$$V = (4/3) \text{ Pi } (\pi) (r)^3$$

Where r is the radius of the particle.
$\pi = 3.14$; $r = (10/2 \text{ nm}) = 5 \text{ nm} = 5 \times 10^{-7} \text{ cm}$.
If we plug in these numbers, $v = 5.23 \times 10^{-19} \text{ cm}^3$
Then $m = (5.23 \times 10^{-19} \text{ cm}^3) (2.648 \text{ gm/cm}^3)$
Or, $m = 1.38 \times 10^{-18} \text{ gm}$

The multifunctional nanoparticle/nanogel product contains two active components, DMDS and first component (*e.g.,* Cu) and a second component (*e.g.,* DMDS). Experimentally, we can load about 33 to 45 wt% of Cu in silica nanoparticle material (measured by ICP-AAS analysis; ICP stands for Inductively Coupled Plasma - Atomic Absorption Spectroscopy). For example, Cu loading is about 33% in Cu loaded silica nanogel material synthesized in acidic ethanol-water mixture containing ethanol (95%) up to 45.5% of total volume. Cu loading is about 45% in Cu loaded silica nanoparticle material synthesized only in acidic water. Roughly one Cu can hold at least one DMDS molecule. These estimates can be applied to the first component and the second component.

An embodiment of the multifunctional silica based nanoparticle and gel are described in PCT Patent Application US 2009/006496 entitled "Silica-based Antibacterial and Antifungal Nanoformulation". In addition, methods of making an embodiment of the multifunctional silica based nanoparticle and gel are described in the aforementioned PCT Patent Application.

Preferably, the precursor material to make the multifunctional silica based nanoparticles and gel can be made by mixing a silane compound (*e.g.,* alkyl silane, tetraethoxysilane, tetramethoxysilane, sodium silicate, or a silane precursor that can produce silicic acid or silicic acid like intermediates and a combination of these silane compounds) with a first component precursor compound in an acid medium (*e.g.,* acidic water) that may contain an alcohol such as ethanol. After mixing for a period of time (*e.g.,* about 30 minutes to a few hours), a mixture including silica nanoparticles loaded with the first component (also referred to as a "loaded silica nanoparticle") is formed. After the loaded silica nanoparticle are formed, the medium can be brought to a pH of about 7 and held for a time period (*e.g.,* a few hours to a day) to form a precursor material that includes a loaded silica nanoparticle gel, where the nanoparticles are inter-connected. This process can be performed using a single reaction vessel or can use multiple reaction vessels.

Once the loaded silica nanoparticle is made, the multifunctional silica based nanoparticles and gel can be formed. The loaded silica nanoparticle can be disposed in a reaction vessel in an aqueous reaction medium (*e.g.,* acidic water) or can be dried and mixed as a powder. The second component (*e.g.,* DMDS) is also added to the reaction vessel that includes the aqueous reaction mixture or the dry precursor material. The ratio of the amount of precursor material and the second component (dry) can be about 1 to 1. This mixture is mixed for a period of time (*e.g.,* from minutes to hours) to form the multifunctional silica based nanoparticles and gel. The multifunctional silica based nanoparticles and gel can be separated (*e.g.,* centrifuge) from the aqueous solution and dried (*e.g.,* air dried). The mixture does not require any additional purification, although further purification and processing can be performed. This process can be performed using a single reaction vessel or can use multiple reaction vessels and can be performed at ambient temperature and pressure.

In a particular embodiment, the second component is DMDS and can be added under mechanical stirring after Cu loaded silica nanoformulation is prepared. However, DMDS can be added anytime during the nanoformulation preparation process. In an embodiment, about 100 micrograms of DMDS is added to about 45 g equivalent of Cu.

In another embodiment of the present disclosure the multifunctional silica based nanoparticles gel can be formed using a second silane compound, where the addition of the second silane compound improves the uniformity of the plant surface coverage. During the step when the silane compound is added, the second silane compound can also be added. The second silane compound can include compounds such as alkyl silanes. The second silane compound can be about 0.01 to 30% or about 10 to 30% weight of the silane compound. The resulting silane mixture can include the first component and/or the second component, such as those described above. The nanoparticle is the same or similar to the nanoparticle described above and herein. It should be noted that an objective in this embodiment is to tailor nanoparticle/nanogel surface hydrophilicity or hydrophobicity to further improve adherence property of nanoformulations. For example citrus leaves are waxy (hydrophobic). To improve adherence of nanoformulation to waxy surface via hydrophobic-hydrophobic interaction, silica nanoparticle/nanogel material can be further modified with a hydrophobic silane reagent such methyl- or propyl- or butyl silane.

As mentioned above, embodiments of the present disclosure are effective for the treatment of diseases affecting plants such as citrus plants and trees. In addition, embodiments of the present disclosure can be effective as a protective barrier against phloem-feeding ACPs as it uniformly covers the plant surface (*e.g.,* leaf surface). In particular, embodiments of the present disclosure can be used to combat citrus canker and greening diseases (HLB). The design of the multifunctional silica based nanoparticle or gel facilitate uniform plant surface coverage or substantially uniform plant surface coverage. In an embodiment, the multifunctional silica based nanoparticle or gel that is applied to plants can have a superior adherence property in various types of exposure to atmospheric conditions such as rain, wind, snow, and sunlight, such that it is not substantially removed over the time frame of the release of the first and/or second components. In an embodiment, the multifunctional silica based nanoparticle or gel has a reduced phytotoxic effect on plants and reduced environmental stress due to minimal Cu content.

Embodiments of the present disclosure can applied on the time frames consistent with the release of the first and second components, and these time frames can include from the first day of application to about a week, about a month, about two months, about three months, about four months, about five months, about six months, about seven month, or about eight months.

A specific embodiment of the multifunctional silica based nanoparticle or gel can include a multifunctional dimethyl disulfide (DMDS) and copper co-loaded silica based nanoparticle or nanogel (DMDS-CuSiNP/NG), which can be used to combat citrus canker and greening diseases is disclosed. When the composition is applied to citrus plants, it is effective for simultaneously controlling citrus canker and citrus greening diseases in a single application during one citrus growing season.

## Comparative Example 1 - CuSiNG Material and Properties

Laboratory-based experimental data confirmed the following: (i) much improved antibacterial properties of CuSiNG material in comparison to controls, Kocide® 3000 (a DuPont product; Cu hydroxide material) and Cu sulfate, (ii) exceptionally strong adherence property to the citrus leaf surface in comparison to controls and (iii) improved surface coverage, uniform throughout, upon spray application. Based on materials characterization data (High Resolution Transmission electron Microscopy and Selected Area Electron Diffraction patterns), it is confirmed that Cu is present in silica nanogel (SiNG) matrix in two different forms and in two different oxidation states, crystalline Cu oxide (Cu+1 state) and amorphous Cu complex (Cu+2 state). The CuSiNG is thus a unique, nanotechnology-enabled engineered nanomaterial. The disc diffusion assay confirmed that the CuSiNG material has the ability to diffuse out from the application location. This diffusion property will have strong impact in protecting rapidly expanding young fruit and leaf surfaces.

## Example 2 - DMDS Loaded CuSiNG in Solution

In the present disclosure, DMDS and Cu co-loaded silica nanogel (DMDS-CuSiNG) based materials and related formulations are prepared. Multidisciplinary research is used to develop and study DMDS loaded materials, conduct a number of laboratory based bioassay to test the efficacy and perform field study to evaluate the efficacy in controlling HLB and citrus canker diseases.

The loading of DMDS in CuSiNG has been studied in solution state (as synthesized CuSiNG liquid formulation) and characterized by the Gas Chromatography-Mass Spectrometry (GC-MS). In solution state, loading of DMDS into CuSiNG material was carried out by directly adding DMDS into the aqueous reaction mixture that contains CuSiNG material. Stirring was continued to ensure uniform mixing of DMDS with the CuSiNG material. The reaction medium composition greatly facilitated direct loading of DMDS into CuSiNG material. After 24 hrs, DMDS-CuSiNG material was centrifuged and air-dried for more than seven days. We were able to smell strong sulphur odor.

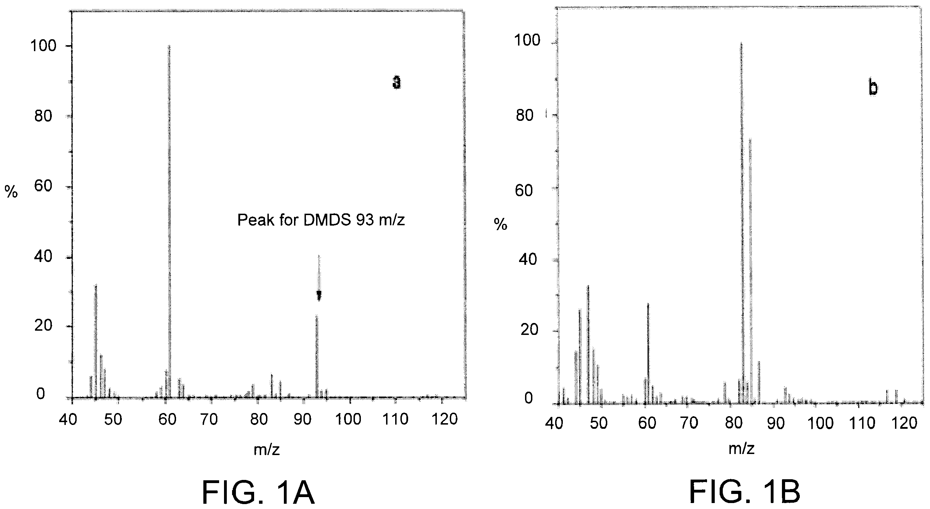

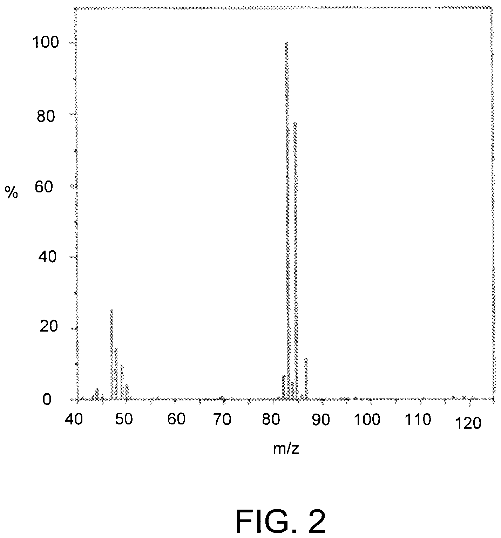

Figure 1a is GC-MS spectra of DMDS used as a control under conditions wherein the silica nanogel (SiNG) does not contain copper. Figure 1b is a GC-MS spectra of DMDS-CuSiNG material. A DMDS odor from the DMDS-CuSiNG powder is discernable even after seven days. For GC-MS sample preparation, spectroscopy grade chloroform was added to the powder and DMDS. GC-MS data of DMDS (control) and chloroform extract of DMDS-CuSiNG are shown in Figure 1a and Figure 1b, respectively. Characteristic molecular peak for DMDS at 93 (m/z) along with other peaks for its fragmented structure is shown in Figure 2 where the GC-MS spectra of DMDS-SiNG material products were found in both cases, confirming the presence of DMDS in DMDS-CuSiNG sample.

Similar experiment was also carried out with SiNG (instead of CuSiNG). After 3 days, we were not able to detect characteristic DMDS odor from DMDS-SiNG material and GC-MS study showed no noticeable DMDS characteristic peaks as shown in Figure 2. These results suggest that $Cu^{2+}$ ions play a critical role with DMDS loading and retention.

Example 3 - Loading of DMDS into CuSiNG in Dry State

Loading of DMDS in dry state using lyophilized CuSiNG powder was carried out by adding DMDS (100 $\mu$l neat) directly to CuSiNG (150 mg of vacuum dried powder) sample in a 20 mL glass vial. For a quick comparison purposes, we took 100 $\mu$l neat DMDS in another 20 mL glass vial (control). Both vials were kept side-by-side inside a laboratory fume hood to allow DMDS to evaporate at the same rate. After 3 days, we were able to smell strong DMDS odor from the DMDS-treated CuSiNG sample only.

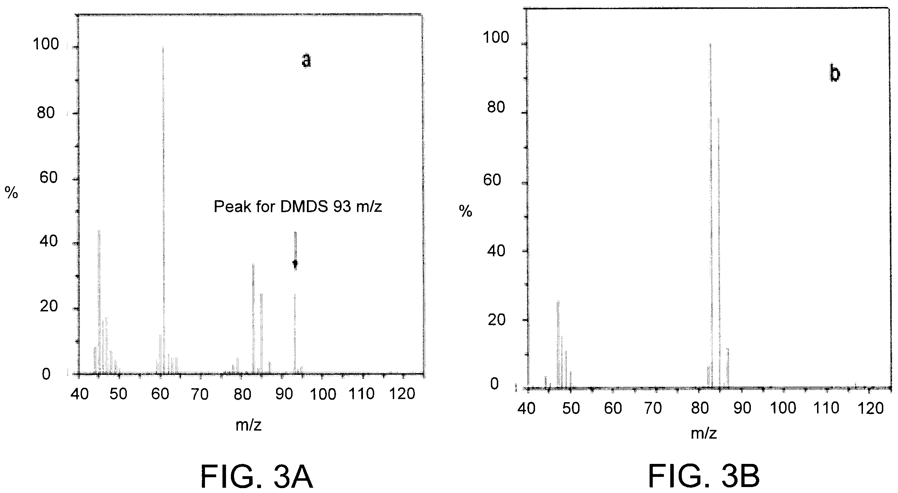

Subsequently, chloroform is added to both vials and results are shown in Figures 3a and 3b. GC-MS spectra of DMDS added to dry CuSiNG lyophilized powder is shown in Figure 3a. In Figure 3b a control extract of DMDS is taken and performed GC-MS. As expected, characteristic DMDS peaks were obtained from DMDS-CuSiNG sample (Figure 3a) and no such peaks from the control (Figure 3b). The above preliminary experiments thus confirmed that CuSiNG material is able to load, retain and slowly release DMDS. A synthesis method for preparation of a silica matrix with embedded metallic particles is reported in U.S. Patent 6,548,264 to Tan et al., U.S. 6,924,116 to Tan et al., and U.S. Patent 7,332,351 to Tan et al. The synthesis of CuSiNG is disclosed in International Patent Application No. PCT/US2009/006496 filed December 10, 2009.

Example 4 - Characterization of DMDS-CuSiNG Material

The following material characterization techniques were used to characterize the DMDS-CuSiNG material of the present disclosure. First a GC-MS study will qualitatively confirm loading of DMDS in CuSiNG and SiNG materials. Second Quartz Crystal Microbalance (QCM) based sensing study will confirm loading and release of DMDS in real-time. Considering practical application of DMDS-CuSiNG material in the field, it is desirable to perform quantitative study to monitor DMDS loading/release processes in real-time. Therefore QCM based sensing technology will be adapted for quantitative measurements of DMDS loading/release and determine kinetics. The sensitivity of QCM technique is reported at the parts per billion level by J.W. Gardner et al, "A brief-history of electronic noses." Sensors and Actuators B-Chemical 1994, 18, (1-3), 211-220. The characterization of DMDS-CuSiNG clarifies the nature of interaction of DMDS with the CuSiNG material. Our goal is to investigate the physico-chemical environment around DMDS and the role of Cu in DMDS adsorption.

Briefly, the experimental setup includes using a sample of CuSiNG material that is spray-coated onto QCM sensor followed by exposure to DMDS in a closed chamber. It is expected that with time the resonating frequency of the QCM will continue to decrease as more and more DMDS is loaded into the CuSiNG material. Once equilibrium is reached, no further frequency drop will take place. The sensor is removed from the chamber and monitoring of the DMDS release process with time is observed. We expect to observe increase in frequency as more and more DMDS is released. Similar experiments will be performed for the SiNG material, as a control.

Example 5 - Role of Cu II ions in DMDS Loading

DMDS loading into SiNG matrix is driven by hydrogen bonding between the silica surface/pores containing silanol (-Si-OH) group (proton donor) and DMDS sulfur atom (proton acceptor), according to R. W. Glass, et al, in "Surface studies of adsorption of sulfur-containing gases at 423 degree K on porous adsorbents. 1. Adsorption of hydrogen sulfide, methanethiol, ethanethiol, and dimethyl sulfide on silica gels." Journal of Physical Chemistry 1973, 77, (21), 2571-2576.

Unlike SiNG, the physico-chemical environment of DMDS in CuSiNG is expected to be somewhat different due to presence of Cu (II) ions. Preliminary results suggest that characteristic odor DMDS does not change over time which indicates that DMDS is nonreactive to Cu(II) ions. However, the DMDS electron-rich sulfur atom has the ability to weakly interact with electron-deficient Cu(II) ion. This could further facilitate adsorption of DMDS into CuSiNG. Thorough FT-IR studies are performed to understand the nature of intermolecular interactions that exist between DMDS and SiNG. In addition, thermogravimetric analysis (TGA), calorimetry and QCM sensing studies to obtain DMDS loading/release characteristics (isotherms) against CuSiNG and SiNG materials will be used to determine the role of Cu II ions in DMDS loading. A comparative isotherm data analysis along with FT-IR analysis will reveal the effect of Cu in DMDS loading/release process.

Example 6 - DMDS Loading into CuSiNG through Nanoscale Manipulaltion

The manipulation of the molecular environment around DMDS at the nanoscale level was conducted to improve DMDS loading efficiency into CuSiNG and SiNG materials.

First hybrid silica nanogel (HSiNG) and Cu loaded HSiNG (CuHSiNG) materials were synthesized. A combination of two inexpensive silica precursors were used during the HSiNG synthesis consisting of a silane based ester and an alkyl based (e.g. methyl or propyl) silane. The rationale of introducing small alkyl chain polymers into the silica matrix is that it will improve interaction with DMDS via intermolecular hydrophobic-hydrophobic interaction.

A series of experiments were performed by varying the ratio of these two silica precursors to optimize loading of both Cu and DMDS into HSiNG material. Both the DMDS-HSiNG and DMDS-HCuSiNG materials are systematically characterized. DMDS loading efficiency to HSiNG and HCuSiNG materials will be evaluated and results will be compared with DMDS-CuSiNG material. For bioassays and field trials, only one DMDS-CuSiNG material that has maximum loading of Cu and DMDS will be selected, the two active components responsible for preventing canker and HLB diseases, respectively.

Characterization: Cu loading efficiency will be quantitatively determined by the atomic absorption spectroscopy (AAS) whereas DMDS loading/release kinetics will be determined by the QCM study. In addition, several material characterization techniques such as TEM/HRTEM (size and morphology), SAED (crystallinity), XPS (identification of Cu oxidation states), XRD (bulk crystallinity), SEM-EDAX (elemental analysis for estimating Cu to Si ratio), BET (surface area/porosity measurements) and FTIR (studying interaction of DMDS as well as Cu with silica matrix) will be used for systematic characterization of HSiNG and CuHSiNG materials. Further improvement of DMDS loading into HSiNG and HCuSiNG is expected due to additional hydrophobic-hydrophobic intermolecular interactions between the methyl groups of DMDS and alkyl groups of silane precursor.

Example 7 - Comparing Efficacy of DMDS-CuSiNG and DMDS-HCuSiNG

Laboratory bioassays for efficacy evaluation of DMDS loaded materials include, disc diffusion assays against X. alfalfae and Olfactometer bioassays against ACPs and the disc diffusion assay. To test anti-bacterial activity of the DMDS-CuSiNG and DMDS-HCuSiNG materials, a known 'disc diffusion assay' method is used.

Briefly, appropriate dilution of overnight grown X. alfalfae culture (200 $\mu$l of about 106 cfu/ml based on dilution of the 0.5 Mc Farland standard) will be spread on nutrient agar plates (90 mm) to achieve a confluent lawn of growth. Comparative analysis of growth inhibition by different concentrations of the test materials (DMDS-CuSiNG and DMDS-HcuSiNG) using DMDS-SiNG and DMDS-HSING as negative controls, and Cu sulfate and Kocide® 3000 as positive controls will be performed to find out the difference in minimum inhibitory concentration ranges under the test conditions. The same volume containing different concentrations of each formulation will be applied per disc to avoid any variation of unequal diffusion perimeter on the agar plate. The level of anti-bacterial ability will be determined by measuring the zone of inhibition after 24 hrs of incubation at 30°C. The minimum inhibitory concentration (MIC) of all the above mentioned compounds, including DMDS-CuSiNG and DMDS-HCuSiNG, will also be determined by spreading appropriate dilution of X. alfalfae culture on nutrient agar plates containing $\log^2$ (two fold) serial dilutions of the test compound(s) to correlate the results of the zone of inhibition in the disc diffusion test and to refine the concentration of DMDS-CuSiNG and DMDS-HCuSiNG necessary for effective killing of the pathogens.

All the test plates will be incubated for longer periods beyond 24 hours to detect any delayed growth of the test organism by the formation of isolated colonies in the zone of inhibition or on the dilution series agar plates at the MIC level. This will tentatively indicate the difference in stability between the DMDS-CuSiNG (or DMDS-HCuSiNG) and Kocide® 3000 or Cu sulfate under the test conditions, which will be an indirect evaluation of the difference in Cu release kinetics of the DMDS-CuSiNG (or DMDS-HCuSiNG) and the Kocide® 3000. We will also generate dose-response plots wherever appropriate. Briefly, dosage of the Cu compound will be varied and the relative response or percentages of disease control to those dosages will be determined.

Statistical analysis: We will test the significance of anti-bacterial activity by different statistical analyses such as ANNOVA discussed in Design and Analysis of Experiments by Hinkelmann, K.; Kempthorne, O., Wiley: 2008; Vol. I and II (Second ed.). Diameter of inhibition zone with all the respective anti-bacterial materials at different concentrations and incubation times will be measured to use in the statistical analysis, assessing the suitability of CuSiNG and H-CuSiNG as a better anti-bacterial material with respect to the controls.

The benefits of applying nanotechnology in citrus research are enormously high. Silica nanogel matrix provides a unique environment for co-hosting Cu and DMDS, making DMDS-CuSiNG nanomaterial multifunctional for combating both citrus canker and greening diseases. Recent studies suggest that silica nanogel is able to slowly spread out from the point of application in moist environment. The impact would be tremendously high as this CuSiNG will form a uniform film-like coating on the surface over time. We identify two major advantages of silica nanogel film; (i) ability to protect rapidly growing young fruit and leaf surfaces and (ii) ability to serve as protective barrier for phloem-feeding ACPs.

The DMDS-CuSiNG technology will offer the following benefits: (a) a two-in-one formulation for combating both citrus canker and greening, (b) superior Cu bioavailability and longevity, (c) sustained release of DMDS for protecting invasion of ACP (primary defense mechanism), (d) robust coating of DMDS-CuSiNG onto leaf surface (secondary defense mechanism against ACP), (e) simple one-pot bulk synthesis technique, (f) inexpensive ingredients (raw material cost ~$3.00 per acre) and (g) multiple applications are not required.

Due to nanoscale engineering, the CuSiNG of the present disclosure has the following advantages over the existing Cu based compounds: uniform coverage of plant surface because of ultra-small particle size, better adherence property due to gel-like nanostructure, sustained (long-term) Cu release profile, better control on Cu release rate (adjustable "soluble" to "insoluble" Cu ratio), more antibacterial/antifungal activity with less amount of Cu content, reduced phytotoxic effect because of adjustable "soluble" to "insoluble" Cu ratio and environment-safe due to less Cu content, no harmful by-product formation, water-based synthesis, utilization of excess CuSiNG as plant nutrient and minimal possibility of having elevated local Cu concentration that could cause environmental toxicity.

The synthesis protocol has the following advantages: (i) simplicity, (ii) water-based, (ii) scalable to field applications, (iii) single-pot synthesis method, requiring no purification steps and (v) concentrated CuSiNG material could be easily diluted for field application. A non-technical person can do this task by adding an appropriate amount of water, thus reducing shipping costs. The method also uses inexpensive raw chemicals and is easily produced in a cost-effective manner.

## Claims

1.  A composition, comprising
    a multifunctional silica based nanoparticle including a first component and a second component, wherein the multifunctional silica based nanoparticle includes a core and shell, wherein the core includes silica loaded with a first type of said first component, wherein the shell includes a first type of said first component and a second type of said first component, wherein the first component functions as an antibacterial, an antifungal, or a combination thereof, and wherein the second component functions as a repellant for insects.

2.  The composition of claim 1, wherein the first component is selected from a copper component, a zinc component, a titanium component, a cerium component, a magnesium component, a zirconium component, a polyethyleneimine (PEI), a fullerene, a carbon nanotube, and a combination thereof; and the second component is a sulfur compound.

3.  The composition of claim 2, wherein the sulfur component is selected from: an alkyl sulfide, an alkyl disulfide, an alkyl trisulfide, an alkyl tetrasulfide, and a combination thereof.

4.  The composition of claim 2, wherein the sulfur component is selected from: dimethyl disulfide (DMDS), dimethyl sulfide, diethyl disulfide, diethyl trisulfide, diethyl tetrasulfide, and a combination thereof.

5.  The composition of claim 4, wherein the first component is a copper component.

6.  The composition of claim 5, wherein the copper component is selected from copper ion, metallic copper, copper oxide, copper oxychloride, copper sulfate, copper hydroxide, and a combination thereof.

7.  The composition of claim 6, wherein the sulfur component is selected from: an alkyl sulfide, an alkyl disulfide, an alkyl trisulfide, an alkyl tetrasulfide, and a combination thereof.

8.  The composition of claim 6, wherein the sulfur component is selected from: dimethyl disulfide (DMDS), dimethyl sulfide, diethyl disulfide, diethyl trisulfide, diethyl tetrasulfide, and a combination thereof.

9.  The composition of claim 6, wherein the sulfur component is dimethyl disulfide (DMDS).

10. The composition of claim 9, wherein the DMDS is attached to the multifunctional silica based nanoparticle via copper ions.

11. A gel comprising:
    a plurality of multifunctional silica based nanoparticles of any one of claims 1 to 10 disposed in an amorphous silica material, wherein the amorphous silica material includes one or both of the first component and the second component.

**12.** The gel of claim 11, further comprising a silane compound.

**13.** The gel of claim 12, wherein the silane compound is selected from the group consisting of: 3-(trihydroxysilyl) propyl methylphosphonate, alkyl silane, tetraethoxysilane, tetramethoxysilane, sodium silicate, a silane precursor that can produce silicic acid or silicic acid like intermediates, and a combination thereof.

**14.** A method for synthesizing a multifunctional silica based nanoparticle gel, the method comprising:

adding a portion of a loaded silica nanoparticle to an aqueous reaction medium to form mixture I wherein the loaded silica nanoparticle is formed by mixing a silane compound with a first component precursor compound in acid medium;
adding a portion of a second component directly to the aqueous reaction medium containing mixture I to form mixture II; and
mixing mixture II to form a multifunctional silica nanoparticle gel that includes multifunctional silica nanoparticles of any one of claims 1 to 10.

**15.** A method for synthesizing a multifunctional silica based nanoparticle gel, the method comprising:

adding a portion of a powdered loaded silica nanoparticle to a reaction vessel, wherein the loaded silica nanoparticle is formed by mixing a silane compound with a first component precursor compound in acid medium;
adding a portion of a second component directly to the powder to form mixture A; and
mixing mixture A to form a multifunctional silica nanoparticle gel that includes multifunctional silica nanoparticles of any one of claims 1 to 10.

**16.** A method of treating, preventing, or both treating and preventing, a disease in a plant species, comprising: administering a composition including multifunctional silica based nanoparticles of claims 1 to 10, multifunctional silica based nanoparticle gel of claims 10 to 13, or a mixture thereof, to a the plant.

**17.** The method of claim 16, wherein the plant treated is a member of the citrus species.

**18.** The method of claim 17, wherein the disease treated is selected from the group consisting of citrus canker, citrus greening, and a combination thereof.

**19.** The method of claim 16, wherein the first component, the second component, or both the first component and second component are released in a sustained manner for treatment of the plant for a time period from the day the composition is administered to about eight months.

**20.** A method for simultaneously treating citrus plants for citrus canker and preventing the invasion of an Asian Citrus Psyllid (ACP) vector that carries the pathogen and spreads the citrus greening disease in citrus plants, comprising the steps of:
administering a composition including multifunctional silica based nanoparticles of claims 1 to 10, multifunctional silica based nanoparticle gel of claims 11 to 13, or a mixture thereof, to a the citrus plant, wherein administering includes substantially covering the leaves and branches of the citrus plant.

**21.** The method of claim 20, further comprising;
allowing the leaching of Cu ions and dimethyl disulfide (DMDS) in a slow-release, non-phytotoxic manner with exposure to atmospheric conditions selected from at least one of rain, wind, snow, and sunlight, wherein a plurality of copper ions and dimethyl disulfide diffuse out from a location of application on a plant tissue surface and cover the exposed tissue surface of rapidly growing fruit and leaves, thereby minimizing the application frequency per growing season, increasing plant surface coverage and longevity of coverage of citrus plants requiring treatment for citrus canker and citrus greening disease.

**22.** The method of claim 20, wherein the silica based formulation further includes a second silane compound to achieve uniform plant surface coverage to modulate copper ion release rate, improve rain-fastness and increase longevity of plant surface coverage.

**23.** The method of claim 22, wherein the second silane compound is selected from the group consisting of: 3-(trihydroxysilyl) propyl methylphosphonate, alkyl silane, tetraethoxysilane, tetramethoxysilane, sodium silicate, a silane precursor that can produce silicic acid or silicic acid like intermediates, and a combination thereof.

24. The method of claim 20, wherein administering includes spraying.

**Patentansprüche**

1. Zusammensetzung, aufweisend ein multifunktionales Siliziumdioxid-basiertes Nanopartikel, umfassend eine erste Komponente und eine zweite Komponente, wobei das multifunktionale Siliziumdioxid-basierte Nanopartikel einen Kern und eine Schale umfasst, wobei der Kern mit einem ersten Typ der ersten Komponente beladenes Siliziumdioxid umfasst, wobei die Schale einen ersten Typ der ersten Komponente und einen zweiten Typ der ersten Komponente umfasst, wobei die erste Komponente als ein antibakterielles Mittel, als ein antimykotisches Mittel, oder als eine Kombination daraus wirkt, und wobei die zweite Komponente als ein Insektenschutzmittel wirkt.

2. Zusammensetzung nach Anspruch 1, wobei die erste Komponente ausgewählt ist aus einer Kupferkomponente, einer Zinkkomponente, einer Titankomponente, einer Cerkomponente, einer Magnesiumkomponente, einer Zirkoniumkomponente, einem Polyethylenimin (PEI), einem Fulleren, einer Kohlenstoffnanoröhre, und einer Kombination davon; und die zweite Komponente ist eine Schwefelkomponente.

3. Zusammensetzung nach Anspruch 2, wobei die Schwefelkomponente ausgewählt ist aus: einem Alkylsulfid, einem Alkyldisulfid, einem Alkyltrisulfid, einem Alkyltetrasulfid, und einer Kombination davon.

4. Zusammensetzung nach Anspruch 2, wobei die Schwefelkomponente ausgewählt ist aus: Dimethyldisulfid (DMDS), Dimethylsulfid, Diethyldisulfid, Diethyltrisulfid, Diethyltetrasulfid, und einer Kombination davon.

5. Zusammensetzung nach Anspruch 4, wobei die erste Komponente eine Kupferkomponente ist.

6. Zusammensetzung nach Anspruch 5, wobei die Kupferkomponente ausgewählt ist aus Kupferionen, metallischem Kupfer, Kupferoxid, Kupferoxychlorid, Kupfersulfat, Kupferhydroxid, und einer Kombination davon.

7. Zusammensetzung nach Anspruch 6, wobei die Schwefelkomponente ausgewählt ist aus: einem Alkylsulfid, einem Alkyldisulfid, einem Alkyltrisulfid, einem Alkyltetrasulfid, und einer Kombination davon.

8. Zusammensetzung nach Anspruch 6, wobei die Schwefelkomponente ausgewählt ist aus: Dimethyldisulfid (DMDS), Dimethylsulfid, Diethyldisulfid, Diethyltrisulfid, Diethyltetrasulfid, und einer Kombination davon.

9. Zusammensetzung nach Anspruch 6, wobei die Schwefelkomponente Dimethyldisulfid (DMDS) ist.

10. Zusammensetzung nach Anspruch 9, wobei das DMDS an das multifunktionale Siliziumdioxid-basierte Nanopartikel über Kupferionen angebunden ist.

11. Gel, aufweisend: eine Vielzahl von multifunktionalen Siliziumdioxid-basierten Nanopartikeln nach einem der Ansprüche 1 bis 10, die in einem amorphen Siliziumdioxid-Material disponiert sind, wobei das amorphe Siliziumdioxid-Material eine oder beide der ersten Komponente und der zweiten Komponente enthält.

12. Gel nach Anspruch 11, weiterhin aufweisend eine Silanverbindung.

13. Gel nach Anspruch 12, wobei die Silanverbindung ausgewählt ist aus der Gruppe bestehend aus: 3-(Trihydroxysilyl)propylmethylphosphonat, Alkylsilan, Tetraethoxysilan, Tetramethoxysilan, Natriumsilikat, einem Silanvorläufer, der Kieselsäure oder kieselsäureartige Zwischenprodukte produzieren kann, und eine Kombination davon.

14. Verfahren zum Synthetisieren eines multifunktionalen Siliziumdioxid-basierten-Nanopartikel-Gels, wobei das Verfahren aufweist:

Zugeben einer Portion eines beladenen Siliziumdioxid-Nanopartikels zu einem wässrigen Reaktionsmedium, um das Gemisch I zu bilden, wobei das beladene Siliziumdioxid-Nanopartikel gebildet wird durch Mischen einer Silanverbindung mit einer erste Komponente-Vorläufer-Verbindung in einem sauren Medium;
Zugeben einer Portion einer zweiten Komponente direkt zu dem wässrigen Reaktionsmedium enthaltend Gemisch I, um Gemisch II zu bilden; und
Mischen des Gemischs II, um ein multifunktionales-Siliziumdioxid-Nanopartikel-Gel zu bilden, das multifunktionale Siliziumdioxid-Nanopartikel nach einem der Ansprüche 1 bis 10 umfasst.

15. Verfahren zum Synthetisieren eines multifunktionalen Siliziumdioxid-basierten-Nanopartikel-Gels, wobei das Verfahren aufweist:

Zugeben einer Portion eines pulverförmigen beladenen Siliziumdioxid-Nanopartikels in ein Reaktionsgefäß, wobei das beladene Siliziumdioxid-Nanopartikel gebildet wird durch Mischen einer Silanverbindung mit einer erste Komponente-Vorläufer-Verbindung in einem sauren Medium;
Zugeben einer Portion einer zweiten Komponente direkt zu dem Pulver, um Gemisch A zu bilden; und
Mischen des Gemischs A, um ein multifunktionales-Siliziumdioxid-Nanopartikel-Gel zu bilden, das multifunktionale Siliziumdioxid-Nanopartikel nach einem der Ansprüche 1 bis 10 umfasst.

16. Verfahren zum Behandeln, Verhindern, oder beidem Behandeln und Verhindern, einer Krankheit in einer Pflanzenart, aufweisend:
Verabreichen einer Zusammensetzung umfassend multifunktionale Siliziumdioxid-basierte Nanopartikel nach einem der Ansprüche 1 bis 10, eines multifunktionalen Siliziumdioxid-basierten-Nanopartikel-Gels nach einem der Ansprüche 10 bis 13, oder einer Mischung davon, an eine Pflanze.

17. Verfahren nach Anspruch 16, wobei die behandelte Pflanze ein Mitglied der Zitrusspezies ist.

18. Verfahren nach Anspruch 17, wobei die behandelte Krankheit ausgewählt ist aus der Gruppe bestehend aus Zitruskrebs, Zitrusgrünung, und einer Kombination davon.

19. Verfahren nach Anspruch 16, wobei die erste Komponente, die zweite Komponente oder sowohl die erste Komponente als auch die zweite Komponente in anhaltender Weise zur Behandlung der Pflanze freigesetzt werden für einen Zeitraum vom Tag, an dem die Zusammensetzung verabreicht wird bis hin zu ungefähr acht Monaten.

20. Verfahren zum gleichzeitigen Behandeln von Zitruspflanzen gegen Zitruskrebs und dem Verhindern der Invasion eines asiatischen Zitrus-Psyllid (ACP)-Vektors, der das Pathogen trägt und die Zitrus-Grünungskrankheit in Zitruspflanzen verbreitet, aufweisend die Schritte:
Verabreichen einer Zusammensetzung umfassend multifunktionale Siliziumdioxid-basierte Nanopartikel nach einem der Ansprüche 1 bis 10, eines multifunktionalen Siliziumdioxid-basierten-Nanopartikel-Gels nach einem der Ansprüche 10 bis 13, oder einer Mischung davon, an die Zitruspflanze, wobei das Verabreichen das substantielle Bedecken der Blätter und Zweige der Zitruspflanze umfasst

21. Verfahren nach Anspruch 20, weiterhin aufweisend:
Gestatten des Auslaugens von Kupferionen und Dimethyldisulfid (DMDS) in einer langsam freisetzenden, nicht phytotoxischen Weise mit Aussetzung unter atmosphärischen Bedingungen, welche ausgewählt sind aus wenigstens einer von Regen, Wind, Schnee und Sonnenlicht, wobei eine Vielzahl von Kupferionen und Dimethyldisulfid von einem Ort der Anwendung auf einer Blattgewebeoberfläche ausdiffundiert und die ausgesetzte Gewebeoberfläche der schnell wachsenden Früchte und Zweige bedeckt, wobei dabei die Verabreichungsfrequenz per Wachstumssaison minimiert wird, die Pflanzenoberflächenbedeckung und die Dauerhaftigkeit der Bedeckung der Zitruspflanzen, die einer Behandlung gegen Zitruskrebs und Zitrusgrünung bedürfen, vergrößert wird.

22. Verfahren nach Anspruch 20, wobei die Siliziumdioxid-basierte Formulierung weiterhin eine zweite Silanverbindung umfasst, um eine gleichmäßige Pflanzenoberflächenbedeckung zu erzielen, um die Kupferionen-Freigaberate zu modulieren, die Regenfestigkeit zu verbessern und die Dauerhaftigkeit der Pflanzenoberflächenbedeckung zu vergrößern.

23. Verfahren nach Anspruch 22, wobei die zweite Silanverbindung ausgewählt ist aus der Gruppe bestehend aus: 3-(Trihydroxysilyl)propylmethylphosphonat, Alkylsilan, Tetraethoxysilan, Tetramethoxysilan, Natriumsilikat, einem Silanvorläufer, der Kieselsäure oder kieselsäureartige Zwischenprodukte produzieren kann, und eine Kombination davon.

24. Verfahren nach Anspruch 20, wobei das Verabreichen das Sprühen umfasst.

**Revendications**

1.  Composition comprenant :
    des nanoparticules à base de silice multifonctionnelles comprenant un premier composant et un second composant, les nanoparticules à base de silice multifonctionnelles comprenant un coeur et une écorce, le coeur comprenant de la silice chargée par un premier type dudit premier composant, l'écorce comprenant un premier type dudit premier composant et un second type dudit premier composant, le premier composant agissant comme un antibactérien, un antifongique ou une combinaison de ceux-ci, et le second composant agissant comme répulsif contre les insectes.

2.  Composition selon la revendication 1, dans laquelle le premier composant est choisi parmi un composant de cuivre, un composant de zinc, un composant de titane, un composant de cérium, un composant de magnésium, un composant de zirconium, une polyéthylèneimine (PEI), un fullerène, des nanotubes de carbone et une combinaison de ceux-ci ; et le second composant est un composé du soufre.

3.  Composition selon la revendication 2, dans laquelle le composant de soufre est choisi parmi : un sulfure d'alkyle, un disulfure d'alkyle, un trisulfure d'alkyle, un tétrasulfure d'alkyle et une combinaison de ceux-ci.

4.  Composition selon la revendication 2, dans laquelle le composant de soufre est choisi parmi : le disulfure de diméthyle (DMDS), le sulfure de diméthyle, le disulfure de diéthyle, le trisulfure de diéthyle, le tétrasulfure de diéthyle et une combinaison de ceux-ci.

5.  Composition selon la revendication 4, dans laquelle le premier composant est un composant de cuivre.

6.  Composition selon la revendication 5, dans laquelle le composant de cuivre est choisi parmi les ions cuivre, le cuivre métallique, un oxyde de cuivre, l'oxychlorure de cuivre, le sulfate de cuivre, l'hydroxyde de cuivre et une combinaison de ceux-ci.

7.  Composition selon la revendication 6, dans laquelle le composant de soufre est choisi parmi : un sulfure d'alkyle, un disulfure d'alkyle, un trisulfure d'alkyle, un tétrasulfure d'alkyle et une combinaison de ceux-ci.

8.  Composition selon la revendication 6, dans laquelle le composant de soufre est choisi parmi : le disulfure de diméthyle (DMDS), le sulfure de diméthyle, le disulfure de diéthyle, le trisulfure de diéthyle, le tétrasulfure de diéthyle et une combinaison de ceux-ci.

9.  Composition selon la revendication 6, dans laquelle le composant de soufre est le disulfure de diméthyle (DMDS).

10. Composition selon la revendication 9, dans laquelle le DMDS est attaché aux nanoparticules à base de silice multifonctionnelles par l'intermédiaire d'ions cuivre.

11. Gel comprenant :
    une pluralité de nanoparticules à base de silice multifonctionnelles de l'une quelconque des revendications 1 à 10 disposées dans une matière de silice amorphe, la matière de silice amorphe comprenant l'un parmi le premier composant et le second composant ou les deux.

12. Gel selon la revendication 11, comprenant en outre un composé silane.

13. Gel selon la revendication 12, dans lequel le composé silane est choisi dans le groupe consistant en : le 3-(trihydroxysilyl)propyl méthylphosphonate, un alkyl silane, le tétraéthoxysilane, le tétraméthoxysilane, le silicate de sodium, un précurseur de silane qui peut produire des intermédiaires acide silicique ou analogues d'acide silicique, et une combinaison de ceux-ci.

14. Procédé de synthèse d'un gel de nanoparticules à base de silice multifonctionnelles, le procédé comprenant :

    ajouter une quantité de nanoparticules de silice chargées à un milieu réactionnel aqueux pour former un mélange I dans lequel les nanoparticules de silice chargées sont formées par mélange d'un composé silane avec un composé précurseur de premier composant en milieu acide ;
    ajouter une quantité d'un second composant directement au milieu réactionnel aqueux contenant le mélange I pour former un mélange II ; et

mélanger le mélange II pour former un gel de nanoparticules de silice multifonctionnelles qui comprend des nanoparticules de silice multifonctionnelles de l'une quelconque des revendications 1 à 10.

15. Procédé de synthèse d'un gel de nanoparticules à base de silice multifonctionnelles, le procédé comprenant :

ajouter une quantité de nanoparticules de silice chargées pulvérulentes dans un récipient de réaction, les nanoparticules de silice chargées étant formées par mélange d'un composé silane avec un composé précurseur de premier composant en milieu acide ;
ajouter une quantité d'un second composant directement à la poudre pour former un mélange A ; et
mélanger le mélange A pour former un gel de nanoparticules de silice multifonctionnelles qui comprend des nanoparticules de silice multifonctionnelles de l'une quelconque des revendications 1 à 10.

16. Procédé de traitement, de prévention ou à la fois de traitement et de prévention, d'une maladie dans une espèce de plante, comprenant :
administrer à la plante une composition comprenant des nanoparticules à base de silice multifonctionnelles des revendications 1 à 10, un gel de nanoparticules à base de silice multifonctionnelles des revendications 10 à 13, ou un mélange de ceux-ci.

17. Procédé selon la revendication 16, dans lequel la plante traitée est un membre des espèces de Citrus.

18. Procédé selon la revendication 17, dans lequel la maladie traitée est choisie dans le groupe consistant en le chancre du Citrus, le verdissement du Citrus et une combinaison de ceux-ci.

19. Procédé selon la revendication 16, dans lequel le premier composant, le second composant, ou à la fois le premier composant et le second composant sont libérés d'une manière entretenue pour le traitement de la plante pendant une période de temps allant du jour où la composition est administrée à environ huit mois.

20. Procédé pour traiter simultanément les plantes de Citrus pour le chancre du Citrus et prévenir de l'invasion d'un vecteur du psylle asiatique du Citrus (Asian Citrus Psyllid-ACP) qui porte le pathogène et propage la maladie du verdissement du Citrus dans les plantes de Citrus, comprenant les étapes de :
administration à la plante de Citrus d'une composition comprenant des nanoparticules à base de silice multifonctionnelles des revendications 1 à 10, d'un gel de nanoparticules à base de silice multifonctionnelles des revendications 11 à 13, ou d'un mélange de ceux-ci, l'administration comprenant un recouvrement substantiel des feuilles et des branches de la plante de Citrus.

21. Procédé selon la revendication 20, comprenant en outre :
permettre la lixiviation d'ions Cu et du disulfure de diméthyle (DMDS) d'une manière non phytotoxique, à libération lente, avec exposition aux conditions atmosphériques choisies parmi au moins l'une de la pluie, du vent, de la neige et de la lumière du soleil, une pluralité d'ions cuivre et le disulfure de diméthyle se diffusant hors d'un emplacement d'application sur une surface de tissu de la plante et recouvrant la surface de tissu exposée de fruits et de feuilles en croissance rapide, rendant ainsi minimale la fréquence d'application par période de croissance, augmentant le recouvrement de surface de la plante et la longévité du recouvrement des plantes de Citrus nécessitant un traitement pour le chancre du Citrus et la maladie de verdissement du Citrus.

22. Procédé selon la revendication 20, dans lequel la formulation à base de silice comprend en outre un second composé silane pour parvenir à un recouvrement de surface de la plante uniforme pour moduler la vitesse de libération des ions cuivre, améliorer la résistance à la pluie et augmenter la longévité du recouvrement de surface de la plante.

23. Procédé selon la revendication 22, dans lequel le second composé silane est choisi dans le groupe consistant en :
le 3-(trihydroxysilyl)propyl méthylphosphonate, un alkyl silane, le tétraéthoxysilane, le tétraméthoxysilane, le silicate de sodium, un précurseur de silane qui peut produire des intermédiaires acide silicique ou analogues d'acide silicique, et une combinaison de ceux-ci.

24. Procédé selon la revendication 20, dans lequel l'administration comprend une pulvérisation.

EP 2 552 196 B1

FIG. 1A

FIG. 1B

FIG. 2

FIG. 3A

FIG. 3B

## REFERENCES CITED IN THE DESCRIPTION

### Patent documents cited in the description

- US 20100015236 A1, MAGDASSI
- US 2009006496 W
- US 6548264 B, Tan
- US 6924116 B, Tan
- US 7332351 B, Tan

### Non-patent literature cited in the description

- **J. M. BOVE.** Huanglongbing: A destructive, newly-emerging, century-old disease of citrus. *Journal of Plant Pathology,* 2006, vol. 88 (1), 7-37
- **A. MORRIS et al.** Economic tradeoffs of citrus greening management. *Citrus Industry,* 2008, vol. 89 (4), 26-28
- **P. L. HOLLIS.** Scientists combining efforts to combat greening in Florida's citrus industry. *Southeast Farm Press,* 2008, vol. 35 (15), 35
- **T. R. GOTTWALD.** The citrus canker epidemic in Florida: The scientific basis of regulatory eradication policy for an invasive species. *Phytopathology,* 2001, vol. 91 (1), 30-34
- **J. H. GRAHAM.** Xauthomouas axonopodis pv. citri: factors affecting successful eradication of citrus canker. *Molecular Plant Pathology,* 2004, vol. 5 (1), 1-15
- **A. K. DAS.** Citrus Canker-A review. *J. Appl. Hort.,* 2003, vol. 5 (1), 52-60
- **KIM, Y. H. et al.** Preparation and characterization of the antibacterial Cu nanoparticle formed on the surface of Si02 nanoparticles. *J. PHYS. CHEM. B,* 2006, vol. 110, 24923-24928
- **J.W. GARDNER et al.** A brief-history of electronic noses. *Sensors and Actuators B-Chemical,* 1994, vol. 18 (1-3), 211-220
- **R. W. GLASS et al.** Surface studies of adsorption of sulfur-containing gases at 423 degree K on porous adsorbents. 1. Adsorption of hydrogen sulfide, methanethiol, ethanethiol, and dimethyl sulfide on silica gels. *Journal of Physical Chemistry,* 1973, vol. 77 (21), 2571-2576
- **HINKELMANN, K. ; KEMPTHORNE, O.** Design and Analysis of Experiments. Wiley, 2008, vol. I and II